# FASCICULE DE BREVET EUROPEEN

(11) **EP 0 766 540 B1**
(45) Date de publication et mention de la délivrance du brevet: **04.08.1999**
(21) Numéro de dépôt: 92921517.6
(22) Date de dépôt: 25.09.1992
(51) Int. Cl.: A61F 2/16

(54) **IMPLANT CRISTALLINIEN**
AUGENLINSENIMPLANTAT
LENS IMPLANT

(30) Priorité: 25.09.1991 FR 9111810
(43) Date de publication de la demande: 09.04.1997
(73) Titulaire: M.N.A.O. MODELISATION NUMERIQUE APPLIQUEE A L'OPHTALMOLOGIE, 75007 Paris (FR)
(72) Inventeur: HANNA, Khalil, F-75004 Paris (FR)
(74) Mandataire: Robert, Jean-Pierre
(86) Numéro de dépôt international: FR9200896
(87) Numéro de publication internationale: WO9305733

(56) Documents cités:
- EP-A- 0 064 770
- EP-A- 0 202 049
- EP-A- 0 337 390
- WO-A-89/07426
- FR-A- 2 530 457
- GB-A- 2 151 371
- US-A- 4 946 469

## Description

La présente invention concerne un implant cristallinien destiné à la correction de l'amétropie due à la perte du dioptre cristallin retiré chirurgicalement.

L'extraction extra capsulaire du cristallin cataracté et l'implantation d'un cristallin artificiel dans le sac capsulaire sont devenues ces dernières années la principale méthode pour soigner la cataracte. Parmi les différentes procédures chirurgicales, la plus utilisée est appelée extraction extra-capsulaire. Elle consiste à retirer le noyau et le cortex cristalliniens après avoir pratiqué une ouverture dans la partie antérieure de la capsule les enfermant. L'extraction est souvent réalisée manuellement par le chirurgien ou au moyen d'une technique dite phacoémulsification. Cette dernière technique présente sur l'extraction manuelle l'avantage d'une petite incision dans la paroi cornéo-sclérale de l'oeil. On sait en effet qu'une petite incision présente moins de risques de complications cliniques ou optiques.

L'inconvénient d'une petite incision réside dans le fait qu'elle n'accepte pas tous les implants et, en particulier les implants rigides dont le diamètre est souvent supérieur à la longueur de l'incision. C'est pour cela que, si le chirurgien décide d'insérer un implant rigide en polyméthylmétacrylate, il procède à l'élargissement de l'incision cornéenne. Mais dans la plupart des cas, pour préserver la petite taille de cette incision cornéo-sclérale, on introduit des implants souples repliables que l'on loge dans la capsule cristallinienne.

Il existe plusieurs types d'implants souples pouvant être introduits pliés, et se dépliant à l'intérieur de la capsule. Leur principal inconvénient réside dans le fait que l'appui de ces implants au niveau de l'équateur de la capsule n'est pas uniforme et cette capsule se ride progressivement, ce qui entraîne une baisse de la vision et demande finalement une intervention secondaire qui consiste à détruire par laser le centre de la partie arrière de la capsule cristallinienne (capsule postérieure). En outre ces implants, du fait même de leur fixation imparfaite dans la capsule, peuvent se déplacer et entraîner une altération de la vision car la zone optique se décentre.

On citera pour illustrer cet art antérieur le document US-A-4 946 469 qui décrit un implant intracapsulaire dans lequel la partie optique est entourée d'une partie de support extrêmement souple pour épouser la capsule postérieure. Cette sorte de jupe très souple ne peut pas donner satisfaction pour le maintien correct de l'implant. En effet on sait qu'après extraction du noyau et des fibres cristalliniens la capsule postérieure tend à se déplacer vers l'avant. Ce mouvement a pour effet de plisser cette capsule, notamment dans la zone optique, ce qui interdit à l'implant selon ce document d'épouser correctement la surface intérieure de la capsule postérieure. Il existe donc des risques de croissance cellulaire entre la capsule et l'implant du genre fibrose qui, au cours du temps vont altérer la qualité de la vue. Cette croissance peut conduire à une déformation et un déplacement de l'implant. Par ailleurs, l'extrême souplesse de la jupe de l'implant ne semble pas pouvoir constituer un moyen de retenue suffisant pour être assuré contre les risques de luxation de l'implant voir de son expulsion à l'extérieur de la capsule. Dans le cas où la lentille est rigide, aucune accommodation n'est possible.

Par ailleurs, il existe un besoin non satisfait par les implants intraocculaires d'aujourd'hui qui réside dans la restauration des facultés d'accommodation d'un patient opéré de la cataracte.

Tous les implants utilisés aujourd'hui ne prennent pas en compte le fait que l'extraction extra-capsulaire peut préserver les facultés d'accommodation du sujet opéré. Il semble en effet que le durcissement du noyau cristallinien soit la cause principale de la presbytie, s'opposant à la déformation de la lentille cristalline naturelle sous l'effet de la contraction et décontraction du muscle ciliaire, plutôt que l'altération de ce muscle ou de la zonule.

Ainsi lorsque le sac capsulaire est préservé lors de l'opération, tous les moyens de l'accommodation le sont également. Certains implants décrits dans la littérature prétendent utiliser la déformation du sac capsulaire pour changer la puissance optique de l'implant. On citera le document EP-A-0 337 390 qui décrit un implant possédant deux parties optiques écartées l'une de l'autre par un élément déformable et dont la distance peut varier avec l'action du muscle ciliaire sur le sac capsulaire par l'intermédiaire des fibres zonulaires. La structure de cet implant est complexe et de manipulation difficile pour sa mise en place dans l'oeil.

La présente invention entend remédier aux inconvénients des implants souples tout en offrant une possibilité d'accommodation, avec des moyens plus simples que ceux jusqu'ici connus.

Il faut tout d'abord rappeler que l'accommodation est pour le cristallin naturel le résultat d'une modification de ses courbures antérieure et postérieure ainsi que de sa position dans le système optique de l'oeil (entre cornée et rétine). Ces modifications résultent de la déformation du sac capsulaire sur lequel agissent les fibres zonulaires le reliant au muscle ciliaire. Lorsque ces fibres sont relâchées, c'est-à-dire lorsque le muscle ciliaire est contracté, le cristallin est dans sa forme et sa position de repos. Les rayons de courbure de ses faces antérieure et postérieure sont à leur plus petite valeur et le cristallin est dans sa position la plus proche de la cornée. L'oeil alors accommode pour la vision de près. En revanche, lorsque les fibres zonulaires sont tendues du fait du relâchement du muscle ciliaire, le sac capsulaire est légèrement étiré vers l'extérieur ce qui a pour effet d'augmenter le rayon de courbure des deux faces du cristallin et de provoquer un léger déplacement de ce dernier vers l'arrière c'est-à-dire en éloignement de la cornée.

L'implant selon l'invention possède les moyens nécessaires à la conversion de l'action du muscle ciliaire en modification de la puissance de sa partie optique.

A cet effet, l'invention a donc pour objet un implant cristallinien en matériau souple comportant une zone optique centrale et des moyens périphériques pour son appui et son centrage dans le sac capsulaire, dans lequel la zone optique est une lentille déformable dont la face postérieure est convexe, les moyens périphériques étant constitués par un bord périphérique dont le diamètre extérieur est sensiblement égal au diamètre équatorial du sac capsulaire au repos et par un dôme de faible épaisseur reliant la lentille au bord périphérique, ce dernier élastiquement déformable formant un tendeur du dôme.

Le rôle premier du bord périphérique est de permettre le déploiement de l'implant à l'intérieur du sac capsulaire après son introduction à l'état replié. Il assure également la bonne mise en place de ce dernier au niveau du méridien du sac puisque sa longueur périphérique sera choisie très voisine de la longueur de ce méridien de sorte que tout risque de luxation ou d'expulsion de l'implant est éliminé.

Pour assurer ce rôle premier, la raideur du bord périphérique peut être conférée par sa forme (par exemple une gouttière de petit rayon de courbure) ou par son épaisseur pour former un bourrelet.

Le dôme forme un voile convexe dont la surface est sensiblement identique à la superficie de la partie correspondante annulaire du sac capsulaire, de sorte que l'implant épouse parfaitement cette face en y exerçant une très légère pression par laquelle la capsule postérieure est maintenue sans plis.

Dans certains modes de réalisation la partie du dôme voisine du bord extérieur peut être plus épaisse que celle voisine de la lentille où il sera avantageux qu'elle soit relativement mince ou de manière équivalente, d'un module d'élasticité différent (par exemple par traitement irradiant d'une matière synthétique afin de créer des zones plus ou moins réticulées) afin de créer une liaison entre voile et lentille plus déformable que ne le permettrait un voile plus épais. Il convient en effet que la tension des fibres zonulaires (lors de la vision de loin avec relâchement du muscle ciliaire), qui tend à déplacer vers l'avant le sac capsulaire, engendre une pression sur la partie centrale de l'implant donc sur la lentille optique afin que cette pression entraîne une modification de ses rayons de courbure telle que la puissance de la lentille soit diminuée comme cela sera décrit plus en détail ci-après, à l'occasion de la description de plusieurs modes possibles de réalisation.

Il sera fait référence aux dessins annexés dans lesquels :
- la figure 1 est une vue en coupe diamétrale d'un premier mode de réalisation de l'invention, logé dans un sac capsulaire à l'état de repos,
- la figure 2 est une demi-vue de l'implant de la figure 1 illustrant l'amplitude des déformations qu'il subit sous l'effet de la traction zonulaire,
- la figure 3 illustre par une vue en perspective avec arraché, un second mode de réalisation de l'invention,
- la figure 4 est une vue en coupe d'un troisième mode de réalisation de l'invention.

L'implant de l'invention est réalisé en un matériau à haut indice de réfraction (par exemple supérieur à 1,50) tel à titre indicatif que les polymères et copolymères fondés sur les polydiphénylsiloxanes qui sont des matériaux de synthèse souples transparents et biocompatibles ou des hydrogels. Cet indice de réfraction élevé permet la construction d'un implant puissant sous une faible épaisseur.

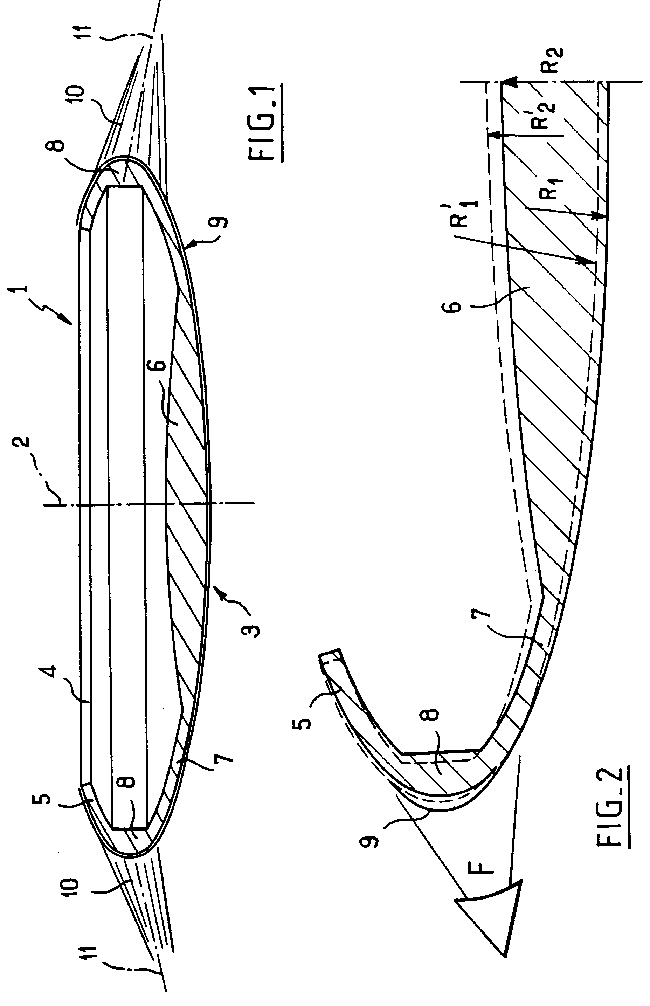

A la figure 1, l'implant l est en forme générale d'ellipsoïde creux de révolution autour de son petit axe 2. L'un des hémisphères 3 possède une paroi complète tandis que la paroi de l'autre possède une ouverture centrale 4 et se limite à une portion annulaire 5 en retour au dessus de la paroi complète 3. La paroi complète 3 comporte une zone optique 6 formant lentille, par exemple biconvexe, située au centre de la paroi. On notera que la face antérieure de cette lentille (celle tournée vers l'ouverture 4) peut être plane ou même concave. Cette lentille se prolonge par un voile annulaire 7 en forme de dôme jusqu'à la zone équatoriale 8 de l'implant où la paroi forme une gouttière et est un peu plus épaisse que le voile pour en constituer un tendeur élastiquement déformable.

Cet implant est destiné à être placé dans le sac capsulaire représenté par le trait 9 de la figure 1.

Les fibres zonulaires 10 sont insérées dans le sac capsulaire au voisinage de l'équateur de ce dernier et leur action est sensiblement dirigée le long d'un cône dont la pointe est tournée vers l'avant et qui est largement ouvert à son sommet. Sur la figure 1, on a représenté les génératrices 11 de ce cône dans le plan de coupe.

Outre son rôle de tendeur du dôme 7 pour le déplier après son insertion dans l'oeil, le bord 8 possède une fonction de maintien de l'implant dans le sac 9 car il aura été choisi de diamètre extérieur sensiblement égal au diamètre équatorial du sac capsulaire 9. Cette raideur née de la gouttière et/ou de la surépaisseur du bord 8 et cette dimension permettent en outre de pratiquer sur le sac une certaine tension qui supprime la formation des plis de la capsule. Le dôme 7 et la partie optique 6 sont donc correctement en appui sur la paroi postérieure de ce sac capsulaire. On sait en effet que ce contact est favorable car il constitue un moyen efficace d'inhibition de la croissance cellulaire dans le sac et donc une prévention du développement d'une fibrose qu'il faudrait ensuite traiter au laser par exemple.

La figure 2 est un schéma qui illustre le comportement de l'implant sollicité par la zonule.

On retrouve sur cette figure la plupart des éléments déjà décrits ci-avant avec les mêmes références. En trait plein, l'implant est représenté dans sa position de repos où il est en contact avec le sac capsulaire 9, les fibres zonulaires étant relâchées. Le rayon de courbure postérieur R₁ de la lentille est ici de 18 millimètres et celui R₂ antérieur est de 30 millimètres.

Sur un modèle mathématique de l'implant on a appliqué sur le sac capsulaire 9, une traction F simulant l'action de la tension des fibres zonulaires, et l'implant s'est déformé jusqu'à sa position représentée en trait pointillé sur la figure 2.

Les rayons de courbure R₁ et R₂ sont devenus R'₁ = 20,5 millimètres et R'₂ = 25,3 millimètres. La zone optique a par ailleurs été déplacée légèrement vers l'avant du sac capsulaire. Cette déformation s'explique par le fait que la paroi postérieure du sac capsulaire sollicitée en traction circulaire par la zonule exerce sur l'implant au niveau de la lentille optique 6 et du dôme 7 une pression vers l'avant alors que le bord 8 plus rigide se déforme très peu (légère rotation). Cette poussée se traduit donc par les variations constatées des rayons de courbure et l'avancée légère de l'implant en direction de la cornée.

On a pu ainsi calculer que pour un indice de réfraction de 1,58, la modification de la forme de la lentille et son déplacement dans le système optique de l'oeil entraînent une diminution de la puissance du cristallin artificiel d'environ 2 à 3 dioptries lors de la vision éloignée et retrouve sa puissance initiale lors de la vision de près (muscle ciliaire contracté et zonule relâchée). Cette constatation est surprenante car, a priori, deux (R₂ et déplacement) des trois paramètres (R₁, R₂ et déplacement) varient dans un sens défavorable à une diminution de puissance.

L'implant selon l'invention est de construction beaucoup plus simple que tous les implants avec faculté d'accommodation de l'art antérieur.

Bien entendu, la forme au repos de cet implant n'est pas limitée à celle représentée aux figures l et 2.

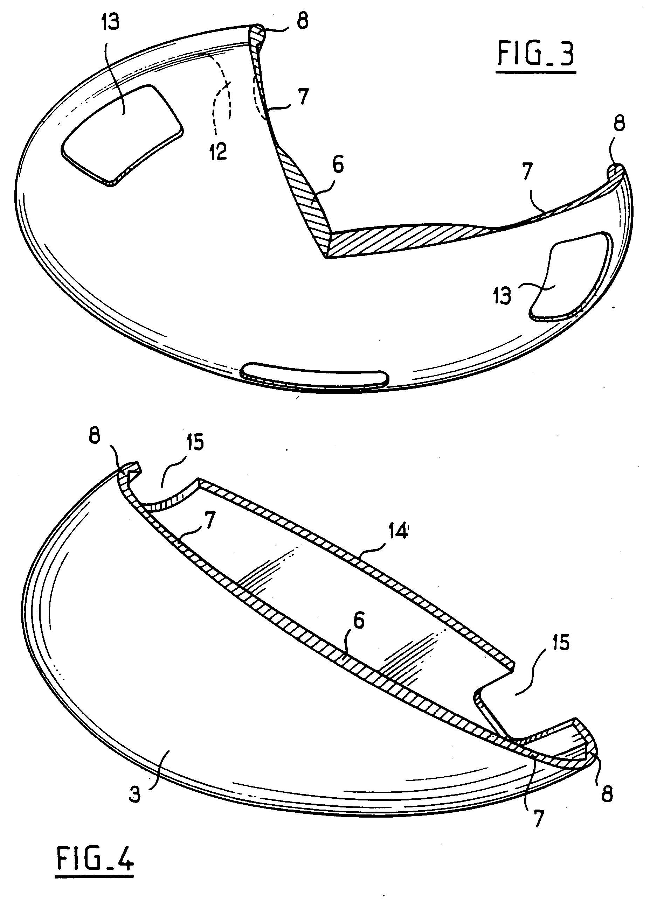

Sur la figure 3 par exemple, il n'existe pas de retour 5 au-delà d'un bourrelet 8 formant le tendeur du dôme 7. En outre ce dôme 7 est d'épaisseur variable en décroissant depuis la périphérie 8 vers la lentille 6. Ainsi la lentille optique dispose-t-elle d'une liberté importante pour être déformée par la pression engendrée par la paroi capsulaire sollicitée par la zonule.

Par ailleurs le dôme peut comporter des éléments de raidissement radiaux 12 qui permettent à la conception d'ajuster la raideur voire la forme du dôme afin qu'elle soit moins déformable au voisinage du bord et de plus en plus souple en direction de la lentille.

Des ouvertures 13 sont pratiquées dans le dôme pour en faciliter la mise en place de la manipulation.

On mentionnera qu'au plan de la forme elle-même, l'implant peut être différent de l'ellipsoïde représenté sans pour autant sortir du cadre de l'invention. On mentionnera une forme dans laquelle la courbure de la face postérieure de l'implant serait beaucoup plus prononcée pour être voisine de celle de la face postérieure du cristallin naturel.

A la figure 4, l'implant représenté comporte une seconde paroi optique 14 à l'avant de la lentille 6 qui peut être une lame à faces parallèles ou une lentille et donc participer à la puissance de l'implant. Cette seconde paroi, qui ferme sur l'avant l'ellipsoïde, est pourvue d'orifices 15 pour la circulation liquide à l'intérieur de l'implant et son pliage. La paroi 14 ne subit pas de déformations du fait de la tension du sac capsulaire car ce sac a été ouvert par sa paroi antérieure qui n'existe donc plus.

## Revendications

1. Implant cristallinien (1) en matériau souple pour mise en place à l'intérieur du sac capsulaire de l'oeil ayant un diamètre équatorial déterminé au repos, l'implant comportant une zone optique centrale (6) et des moyens pour son appui et son centrage dans ce sac capsulaire (9) comprenant un dôme (7) de faible épaisseur reliant la lentille (6) à un bord périphérique (8), l'implant étant constitue par un corps creux sensiblement ellipsoïdal dont l'une des parois porte la lentille (6) et le dôme (7), dont la zone équatoriale forme le bord périphérique (8) susdit et dont l'autre paroi est pourvue d'au moins une ouverture, caractérisé en ce que la zone optique centrale est une lentille déformable, le bord périphérique étant d'épaisseur plus importante que celle du dôme (7) pour former tendeur de ce dernier et étant de diamètre extérieur sensiblement égal audit diamètre équatorial du sac capsulaire au repos.

2. Implant selon la revendication 1 caractérisé en ce que l'épaisseur du dôme (7) varie de manière décroissante entre le bord (8) et la lentille (6).

3. Implant selon la revendication 1 ou la revendication 2 caractérisé en ce que le dôme est pourvu de raidisseurs radiaux (12) au voisinage de son bord périphérique (8).

## Claims

1. A lens implant (1) of flexible material for implanting in an eye capsular sac having determined equatorial diameter at rest, the implant including a central optical zone (6) and means for supporting and centering it in this capsular sac (9) comprising a thin dome (7) connecting the lens (6) to a peripheral edge (8), the implant (1) being constituted by a substantially ellipsoidal hollow body one of whose walls carries the lens (6) and the dome (7), whose equatorial zone forms said peripheral edge (8) and its other wall (14) is provided with at least one opening, characterized in that the central optical zone is a deformable lens, the peripheral edge being thicker than the dome (7) to constitute a tensioning member for the dome and having an outside diameter substantially equal to the equatorial diameter of the capsular sac at rest.

2. An implant according to claim 1, characterized in that the thickness of the dome (7) tapers from the edge (8) towards the lens (6).

3. An implant according to claim 1 or claim 2, characterized in that the dome is provided with radial stiffeners (12) in the vicinity of its peripheral edge (8).

## Patentansprüche

1. Augenlinsenimplantat (1) aus weichem Material zum Einsetzen in den Linsenbeutel des Auges, der im Ruhezustand einen vorgegebenen Äquatorialdurchmesser hat, wobei das Implantat eine optische Zentralzone (6) und Mittel zu seiner Anlage und zu seiner Zentrierung in dem Linsenbeutel (9) hat mit einem gewölbten Ringbereich (7) geringer Dicke, welcher die Linse (6) mit einem Umfangsrand (8) verbindet, und wobei das Implantat aus einem im wesentlichen ellipsoiden Hohlkörper besteht, dessen eine Wand die Linse (6) und den gewölbten Ringbereich (7) umfaßt, dessen äquatoriale Zone den Umfangsrand (8) desselben bildet und dessen andere Wand mit mindestens einer Öffnung versehen ist, dadurch **gekennzeichnet**, daß die optische Zentralzone eine verformbare Linse ist, wobei der Umfangsrand eine gegenüber dem gewölbten Ringbereich (7) wesentlich größere Dicke hat, um einen Spanner für letzteren zu bilden, und wobei der Außendurchmesser des Umfangsrandes im wesentlichen gleich des Äquatorialdurchmessers des Linsenbeutels im Ruhezustand desselben ist.

2. Implantat nach Anspruch 1, dadurch **gekennzeichnet**, daß die Dicke des gewölbten Ringbereiches (7) zwischen dem Rand (8) und der Linse (6) in abnehmender Weise variiert.

3. Implantat nach Anspruch 1 oder 2, dadurch **gekennzeichnet**, daß der gewölbte Ringbereich nahe seinem Umfangsrand (8) mit radialen Versteifungsrippen (12) versehen ist.
